# EUROPEAN PATENT APPLICATION

(11) **EP 2 629 095 A1**
(43) Date of publication of application: **21.08.2013**
(21) Application number: 12001085.5
(22) Date of filing: 17.02.2012
(51) Int. Cl.: G01N 33/543, G01N 33/569, G01N 33/58

(54) **Detection of mycobacterial protein targets using quantum dots (QDs) and immunomagnetic seperation**

(71) Applicant: Biosure R & T Cell Co., Athens (GR); Ikonomopoulos, John, 152 34 Halandri (GR)
(72) Inventor: Gazouli, Maria, 116-33 Athens (GR); Liandris, Emmanouil, 113-61 Athens (GR); Ikonomopoulos, John, 152-34 Halandri, Athens (GR); Tachtsidis, Ilias, London SE25 5HJ (GB); Goutas, Nicholaos, 152-37 Filothei, Athens (GR)
(74) Representative: Malamis, Alkisti-Irene

(57) **Abstract**

The invention describes the detection of members of the Mycobacterium genus by use of a technique for detecting mycobacterial antigens using quantum dot (QD) labels and immunomagnetic separation with antibody coated Magnetic Beads (MBs). This allows rapid and direct detection of the main mycobacterial pathogens of the *Mycobacterium tuberculosis* complex (MTC) in clinical samples, in a way that is highly specific, very easy to perform, and requires minimum infrastructure and expertise.

## Description

### Field of the invention

The general technical field of the invention relates to the detection of members of the Mycobacterium genus.

### Background to the invention

Pathogens of the genus *Mycobacterium* are a major cause of morbidity and mortality in humans and animals worldwide. Tuberculosis results nowadays to millions of deaths or disabilities each year, especially in poorer areas of the planet according to WHO reports, and this more than 100 years after the first description by Koch of the bacteria responsible. The problem is exacerbated by the AIDS epidemic that increases disease incidence in developed countries too. (www. who.org/tb/en).

Concerning humans, in addition to tuberculosis, exposure to mycobacteria has also been linked to the pathogenesis of sarcoidosis and Crohn's disease that affect millions of people in Europe only. The disease threat posed by the pathogens of the genus *Mycobacterium* is not unique to humans. Non-human primates can be infected by *Mycobacterium tuberculosis,* while cattle and other ruminants are natural hosts of the closely-related species M. bovis, which causes substantial financial loss in many regions of the world. The M. *avium* complex affects birds and mammals within this complex M. *avium* subsp *paratuberculosis* (MAP) causes paratuberculosis in ruminants.

### Summary of the invention

The invention provides use of a technique for detecting mycobacterial antigens using quantum dot (QD) labels and immunomagnetic separation with antibody coated Magnetic Beads (MBs). This allows rapid and direct detection of the main mycobacterial pathogens of the *Mycobacterium tuberculosis* complex (MTC) in clinical samples, in a way that is highly specific, very easy to perform, and requires minimum infrastructure and expertise.

Accordingly the invention provides a method of determining the presence of mycobacterial antigens in a sample comprising using a capture antibody conjugated to Magnetic Beads (MBs), which is a mycobacterial polyclonal antibody.A sample is added, and any antigen present binds to capture antibody. The antigen bound by capture antibody is then concentrated by the use of a magnetic device and detected with a biotin conjugated second antibody, which is a mycobacterial monoclonal antibody followed by an anti-mouse IgG biotin conjugated antibody. Detection is performed by addition of streptavidin linked Quantum Dot (QD); and wherein the determination of whether the sample comprises a mycobaterial protein is carried out by detection of a change in fluorescence.

### Detailed description of the invention

The invention allows detection of mycobacterial antigens , from the *Mycobacterium tuberculosis* complex (MTB),
QDs are semiconductor nanocrystals whose photoluminescence emission wavelength is proportional to the size of the crystal. The emission spectra of the QDs are narrow which allows multiwavelength labeling and effectively multi-target labeling with different sizes of QDs with little overlap. Their outer surface can be readily conjugated to organic molecules, resulting in a spectrum of biologically compatible labels that are excited with a single wavelength (Bruchez et al., 1998, Chan and Nie, 1998).
QDs are already integrated in research and routine diagnostic applications. (Parungo et al., 2005). Targeting and imaging of cancerous cells has been used by several research groups both in vivo (Gao et al., 2004, Stroh et al., 2005) and *in vitro* (Tokumasu et al., 2005). Selective distraction of these cells has also been exploited and offers a fascinating prospect for treatment (Sonvico et al., 2005). Recently, QDs have been used for the detection of several different bacterial species providing also information on their viability (Kloepfer et al., 2003, Su and Li, 2004, Dwarakanath et al., 2004).
QDs are highly sensitive biosensors, since they already constitute the basis of a very innovative diagnostic approach. The ability to detect very small amounts of the QDs can decrease dramatically the lower detection limit of the diagnostic assays in which they are incorporated, allowing direct, even in-field detection of microbial nucleotide sequences, without the need for amplification. QDs are very promising as they could be used for the simultaneous quantitative detection of different targets. A great number of samples can be processed at the same time while the use of portable devices could permit the in-field detection of the QDs.

In one embodiment of the method it is carried out to diagnose infection of an individual suspected to have a mycobacterial infection, wherein the individual is preferably human. The invention provides a method of determining the presence of mycobacterial antigens in a sample comprising using a capture antibody, which is a mycobacterial polyclonal antibody.

In one embodiment, this determination is carried out by adding a sample, and any antigen present binds to capture antibody conjugated to MBs, wherein the antigen bound by capture antibody is then isolated and concentrated by a magnetic device and detected with a second antibody which is a mycobacterial monoclonal antibody, different from the one used for capture, followed by an anti-mouse IgG biotin conjugated antibody and wherein detection is performed by addition of streptavidin linked Quantum Dot;

In the embodiment described above detection of whether the first antibody has bound to a protein in the sample will be done by measuring the presence of the quantum dot (which is now effectively bound to the protein via antibodies). This may be achieved by detection of a change in fluorescence, for example using UV light detection or using a fluorescence plate detector.

The invention may be carried out to diagnose whether or not an individual has mycobacterial infection, in which cases the sample will be from the relevant individual. The individual is typically a mammal, such as a human. The individual may or may not have symptoms of a mycobacterial disease. The sample generally comprises clinical samples adeguate for culture.

The capture antibody is a biotinilated polyclonal antibody produced in rabbit against M. *tuberculosis* PPD. This antibody may specifically bind a protein of M. *tuberculosis* complex.,.

The second antibodies are typically monoclonal antibodies. An antibody used in the method of the invention may either be a whole antibody or a fragment thereof which is capable of binding the same epitope. Typically the antibody is a mammalian antibody, such as a primate, human, rodent (e.g. mouse or rat), rabbit, ovine, porcine, equine or camel antibody. The antibody can be any class or isotype of antibody, for example IgM, but is preferably IgG.

The fragment of whole antibody that can be used in the method comprises an antigen binding site, e.g. Fab or F(ab)₂ fragments. The whole antibody or fragment may be associated with other moieties, such as linkers which may be used to join together 2 or more fragments or antibodies. Such linkers may be chemical linkers or can be present in the form of a fusion protein with the fragment or whole antibody. The linkers may thus be used to join together whole antibodies or fragments which have the same or different binding specificities.

The invention also provides a kit for carrying out the method comprising 1 or 2 different first antibodies or second antibodies as defined herein, and optionally also instructions for carrying out the method. The kit may also comprise a means to detect quantum dots.

Any of the following antibodies (which are produced by their respective hybridomas) may be used as first antibody:
*M.tuberculosis* HBHA
   1B11.1
   4A8
   1G10
   4C10
   3H4
   5F3

Typically, a QD is a semiconductor nanocrystal, whose radius is smaller than the bulk exciton Bohr radius, which constitutes a class of materials intermediate between molecular and bulk forms of matter. QDs are typically formed from inorganic, crystalline semiconductive materials and have unique photophysical, photochemical, and nonlinear optical properties arising from quantum size effects.

A QD may exhibit a number of unique optical properties due to quantum confinement effects. For example, QDs possess strong fluorescence, photostability against bleaching and physical environments such as pH and temperature, and optical tunability, overcoming many of the shortcomings apparent with organic fluorophores.

QDs may be formed from an inner core of one or more first semiconductor materials that optionally may be contained within an overcoating or "shell" of a second semiconductor material. A QD core surrounded by a semiconductor shell is referred to as a "core/shell" QD. The optional surrounding shell material will preferably have a bandgap energy that is larger than the bandgap energy of the core material and may be chosen to have an atomic spacing close to that of the core substrate. Suitable semiconductor materials for the core and/or the optional shell include, but are not limited to, the following: materials comprised of a first element selected from Groups 2 and 12 of the Periodic Table of the Elements and a second element selected from Group 16 (e.g., ZnS, ZnSe, ZnTe, CDs, CdSe, CdTe, HgS, HgSe, HgTe, MgS, MgSe, MgTe, CaS, CaSe, CaTe, SrS, SrSe, SrTe, BaS, BaSe, BaTe, and the like); materials comprised of a first element selected from Group 13 of the Periodic Table of the Elements and a second element selected from Group 15 (e.g., GaN, GaP, GaAs, GaSb, InN, InP, InAs, InSb, and the like); materials comprised of a Group 14 element (Ge, Si, and the like); materials such as PbS, PbSe and the like; and alloys and mixtures thereof. As used herein, all reference to the Periodic Table of the Elements and groups thereof is to the IUPAC system for numbering element groups, as set forth in the Handbook of Chemistry and Physics, 81st Edition (CRC Press, 2000). QDs may be made using techniques known in the art. See, e.g., U.S. Pat. Nos. 6,048,616; 5,990,479; 5,690,807; 5,505,928; and 5,262,357. QDs used in the present disclosure may absorb a wide spectrum of light, and may be physically tuned with emission bandwidths in various wavelengths. See, e.g., Badolato, et al., Science 208:1158-61 (2005). For example, the emission bandwidth may be in the visible spectrum (e.g., from about 3.5 to 7.5 [mu]m), the visible-infrared spectrum (e.g., from about 0.1 to about 0.7 [mu]m), or in the near-infrared spectrum (e.g., from about 0.7 to about 2.5 [mu]m). QDs that emit energy in the visible range include, but are not limited to, CdS, CdSe, CdTe, ZnSe, ZnTe, GaP, and GaAs. QDs that emit energy in the blue to near-ultraviolet range include, but are not limited to, ZnS and GaN. QDs that emit energy in the near-infrared range include, but are not limited to, InP, InAs, InSb, PbS, and PbSe. QDs with emission spectra in the near- infrared spectrum may be particularly suited for probes used in certain imaging applications. For example, such QDs may be suited for in vivo imaging, among other things, due to tissue's high optical transmissivity in the near-infrared spectrum.For attachment, the QD may comprise a functional group or attachment moiety. One example of such a QD that has a functional group or attachment moiety is a QD with a carboxylic acid terminated surface, such as those commercially available though, for example, Quantum Dot, Inc., Hayward, CA. The QD is preferably a CdSe QD.

The presence of the QD will generally be carried out by a detector capable of detecting fluorescence from a QD. To detect fluorescence, the detector should be positioned in relation to the sample such that fluorescence can be detected, and the detector should be adapted to detect fluorescence. One example of a suitable detector is a fluorimeter. In some embodiments, the system may further comprise a source of electromagnetic radiation, for example, an excitation monochromator. An excitation monochromator may be used to excide the probe at a specific wavelength to ensure the quality of emission detection. Specific mycobacterial proteins are mentioned herein. In one embodiment the first antibody specifically binds homologues of any of these specific proteins. Such homologues typically have at least 70% homology, preferably at least 80, 90%, 95%, 97% or 99% homology, for example over a region of at least 15, 20, 30 or more contiguous amino acids, or even across the entire length of the original sequence. The homology may be calculated on the basis of amino acid identity (sometimes referred to as "hard homology").

For example the UWGCG Package provides the BESTFIT program which can be used to calculate homology (for example used on its default settings) (Devereux et al (1984) Nucleic Acids Research 12, p387-395). The PILEUP and BLAST algorithms can be used to calculate homology or line up sequences (such as identifying equivalent or corresponding sequences (typically on their default settings), for example as described in Altschul S. F. (1993) J Mol Evol 36:290-300; Altschul, S, F et al (1990) J Mol Biol 215:403-10.

Software for performing BLAST analyses is publicly available through the National Center for Biotechnology Information (This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands. The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

The homologous sequence typically differs by 1, 2, less than 5, less than 10 or less than 15 mutations (each of which may be a substitution, deletion or insertion of a nucleotide). These mutations may be measured across any of the regions mentioned above in relation to calculating homology.

The following Example illustrates the invention.

### Example

Here we report the development a fluorescence immunoassay using a QD-streptavidin approach and Magnetic Beads that is used to show the binding of anti-mycobacterial monoclonal antibodies to mycobacterial antigens in biological samples. QDs with a streptavidin bridge, immobilized on their surface via electrostatic interactions, were purchased from Invitrogen. Polyclonal biotinylated antibodies used to interact with Magnetic Beads were purchased from Acris. MAgnetic Beads coated with Steeptavidin were purchased by Invitrogen:

### MTB monoclonal antibodies

Proteins of *M.tuberculosis* (MTB) used for monoclonal antibodies production were: **HBHA :** heparin-binding hemagglutinin adhesin, a ∼30 kDa recombinant antigen required for extra pulmonary dissemination.

Mycobacterial antigens, were constructed as 6xhistidine (6xHis)-tagged recombinant proteins. The recombinant protein was over expressed by using 1 mM IPTG and purified by nickel chromatography or by maltose affinity chromatography, following standard protocols.

### Monoclonal selected antibodies were:

### Methylated HBHA (6 monoclonal antibodies)

### Identification of Mycobacterial antigens:

### QDs Protocol (by using a secondary antibody biotin conjugated)

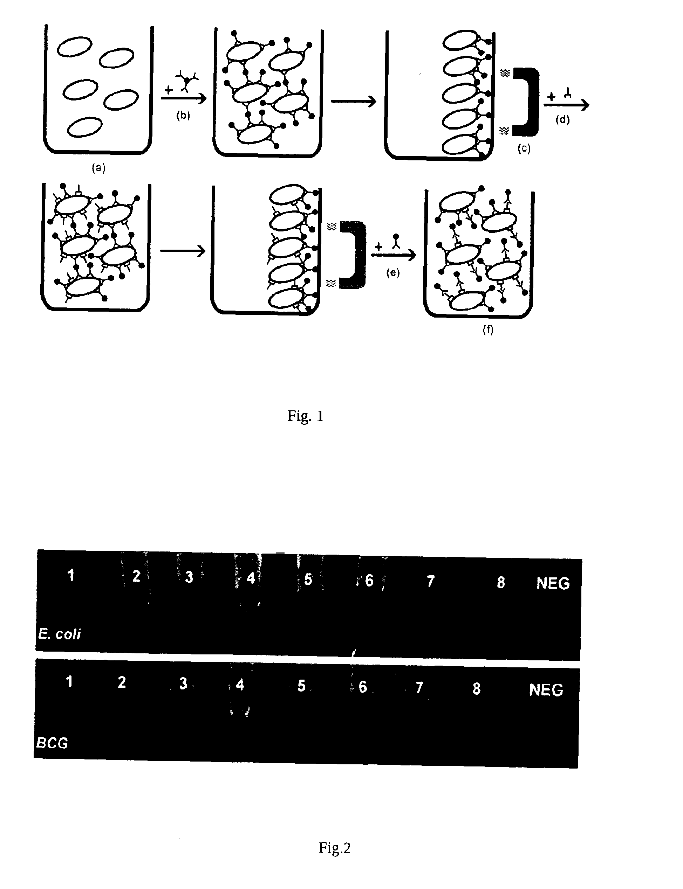

1. The principle of the assay is based upon the separation of bacterial cells using MBs coupled with genus-specific polyclonal as well as the mouse monoclonal antibodies mentioned above. These complexes are then tagged with anti-mouse biotinylated antibody and finally streptavidin-conjugated QDs which lead to the detection of a fluorescent signal (Figure 1).
2. 1 ml of sample for example diluted sputum or bronchoalveolar lavage are coupled at first with 40 µl of genus specific polyclonal antibody conjugated MBs
3. 1.2 mg (200 ng each) of HBHA monoclonal antibody is added
4. 10 µl (0.2 mg/ml) of an anti-mouse biotinylated antibody
5. Finally 40 µl of streptavidin coated QDs are added.
6. For each step samples are incubated at 37°C for 20 min with gentle agitation, separated by the matrix with the aid of a magnetic device washed once with PBS BSA 0.1% and then re-suspended in 200 ml PBS 0.1% BSA.
7. For the direct visual observation of fluorescence, samples are transferred on a UV transilluminator with UV emission at 312 nm.
8. Fluorescence may also be detected with a spectrofluorometer and with an epifluorescence microscope with appropriate filters

### Representative results:

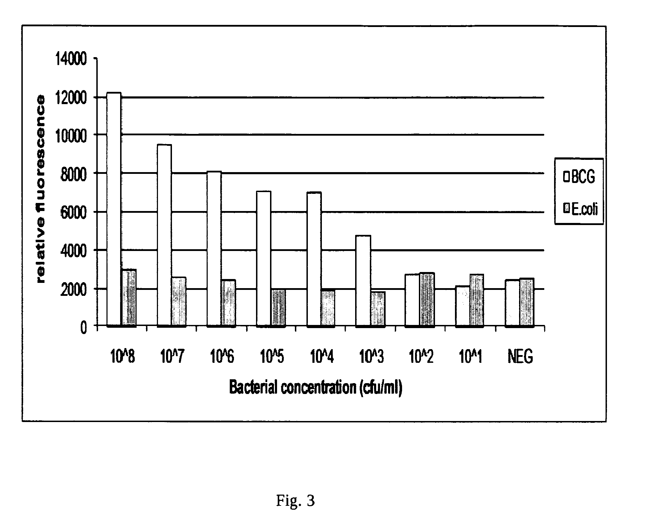

1. Two series of ten-fold dilutions ranging from 10⁸ to 10 cells/ml were prepared by suspending a few fresh colonies grown on solid media in sterile screw cup glass tubes containing PBS The number of bacteria in the suspension was determined with the aid of a Petroff Hauser counting chamber.
2. Fluorescence detection was performed by UV visualization (Fig 2) and fluorimentry with a Tecan instrument (gain optimal). See Fig 3

### Detailed description of the Figures:

Figure 1 shows that bacterial cells (a) are separated from the matrix using MBs coupled with genus specific polyclonal antibodies (b) and a magnetic device (c). These complexes are then tagged with anti-HBHA (d) and anti-mouse biotynylated antibody and streptavidin-conjugated QDs (e) which lead to the detection of a fluorescent signal (f).
Figure 2 shows results recorded by the proposed method for E. coli and M. bovis BCG serial dilutions ranging from 108to 101 cfu/ml (numbers 1-8) and negative controls. Fluorescence is evident in BCG samples with concentration up to 104 cfu/ml.
Figure 3 shows relative fluorescence values of BCG and *E*. *coli* serial dilutions

### Literature

Bruchez, M. Jr., Moronne, M., Gin, P., Weiss, S., Alivisatos, A.P., 1998. Semiconductor nanocrystals as fluorescent biological labels. Science. 281, 2013-2016.
Chan, W.C., Nie, S., 1998. Quantum dot bioconjugates for ultrasensitive nonisotopic detection. Science. 281, 2016-2018.
Cole, S.T., Brosch, R., Parkhill, J., Garnier, T., Churcher, C., Harris, D., Gordon, S.V., Eiglmeier, K., Gas, S., Barry, C.I., Tekaia, F., Badcock, K., Basham, D., Brown, D., Chillingworth, T., Connor, R., Davies, R., Devlin, K., Feltwell, T., Gentles, S., Hamlin, N., Holroyd, S., Hornsby, T., Jagels, K., Krogh, A., McLean, J., Moule, S., Murphy, L., Oliver, K., Osborne. J., Quail, M.A., Rajandream, M.A., Rogers, J., Rutter, S., Seeger, K., Skelton, J., Squares, R., Squares, S., Sulston, J.E., Taylor, K., Whitehead, S., Barrell, B.G., 1998. Deciphering the biology of Mycobacterium tuberculosis from the complete genome sequence. Nat. 393, 537-544.
Dwarakanath, S., Bruno, J.G., Shastry, A., Phillips, T., John, A.A., Kumar, A., Stephenson, L.D., 2005. Quantum dot-antibody and aptamer conjugates shift fluorescence upon binding bacteria. Biochem. Biophys. Res. Commun. 325, 739-743.
Gao, X., Cui, Y., Levenson, R.M., Chung, L.W., Nie, S., 2004. In vivo cancer targeting and imaging with semiconductor quantum dots. Nat. Biotechnol. 22, 969-976.
Kloepfer, J.A., Mielke, R.E., Wong, M.S., Nealson, K.H., Stucky, G., Nadeau, J.L., 2003. Quantum dots as strain- and metabolism-specific microbiological labels. Appl. Environ. Microbiol. 69, 4205-4213.
Parungo, C.P., Ohnishi, S., Kim, S.W., Kim, S., Laurence, R.G., Soltesz, E.G., Chen, F.Y., Colson, Y.L., Cohn, L.H., Bawendi, M.G., Frangioni, J.V., 2005. Intraoperative identification of esophageal sentinel lymph nodes with near-infrared fluorescence imaging. J. Thorac. Cardiovasc. Surg. 129, 844-850.
Sonvico, F., Dubernet, C., Colombo, P., Couvreur, P., 2005. Metallic colloid nanotechnology, applications in diagnosis and therapeutics. Curr. Pharm. Des. 11, 2095-2105.
Stroh, M., Zimmer, J.P., Duda, D.G., Levchenko, T.S., Cohen, K.S., Brown, E.B., Scadden, D.T., Torchilin, V.P., Bawendi, M.G., Fukumura, D., Jain, R.K., 2005. Quantum dots spectrally distinguish multiple species within the tumor milieu in vivo. Nat. Med. 11, 678-682.
Su, X.L., Li, Y., 2004. Quantum dot biolabeling coupled with immunomagnetic separation for detection of Escherichia coli O157:H7. Anal. Chem. 76, 4806-4810.
Tokumasu, F., Fairhurst, R.M., Ostera, G.R., Brittain, N.J., Hwang, J., Wellems, T.E., Dvorak, J.A., 2005. Band 3 modifications in Plasmodium falciparum-infected AA and CC erythrocytes assayed by autocorrelation analysis using quantum dots. J. Cell. Sci. 118, 1091-1098.

## Claims

1. A method of determining the presence of mycobacterial antigens in a sample comprising using a capture antibody conjugated to Magnetic Beads, which is a mycobacterial polyclonal antibody.
A sample is added, and any antigen present binds to capture antibody. The antigen bound by capture antibody is then separated by the matrix with the aid of a magnetic device and detected with a second antibody which is a mycobacterial monoclonal antibody, followed by an anti-mouse IgG biotin conjugated antibody. Detection is performed by addition of streptavidin linked Quantum Dot and wherein optionally the determination of whether the sample comprises a mycobaterial antigen is carried out by detection of a change in fluorescence.

2. A method according to claim 1 which is carried out to diagnose infection of an individual suspected to have a mycobacterial infection, wherein the individual is preferably human,

3. A method according to any one of the preceding claims wherein the detection antibody specifically binds to heparin-binding hemagglutinin adhesin, methylated heparin-binding hemagglutinin adhesin.

4. A method according to any one of the preceding claims wherein the detection antibody specifically binds a protein of M. *tuberculosis,* wherein the protein is optionally methylated heparin-binding hemagglutinin adhesin.

5. A method according to any one of the preceding claims wherein the detection antibodies are monoclonal antibodies, or are in the form of serum.

6. A method according to any one of the preceding claims wherein 2, 3, 4 or more different first antibodies are used, optionally labeled with different quantum dots labels.

7. A method according to claim 6 in which the different quantum dots each have a different emission spectrum.

8. A method according to any one of the preceding claims in which the sample comprises clinical samples adeguate for mycobacterial culture and/or is from an individual who does not have any symptoms of mycobacterial infection or disease.

9. A method according to any one of the preceding claims wherein the quantum dots are detected using UV light or a fluorescent plate reader.

10. A kit for carrying out the method of any one of claims 1 to 12 comprising streptavidin bound to a quantum dot and either (i) 1, 2 or 3 different first antibodies as defined in claim 1 and 1, 2 or 3 second antibodies as defined in claim 1, wherein the second antibodies are bound to biotin,wherein the kit optionally also comprises instructions for carrying out the method.
